# EUROPÄISCHE PATENTSCHRIFT

(11) **EP 0 662 803 B1**
(45) Veröffentlichungstag und Bekanntmachung des Hinweises auf die Patenterteilung: **23.04.1997**
(21) Anmeldenummer: 93920805.4
(22) Anmeldetag: 23.09.1993
(51) Int. Cl.: A61B 17/36

(54) **VERFAHREN UND VORRICHTUNG ZUR FORMKORREKTUR EINER LINSE**
PROCESS AND DEVICE FOR CORRECTING THE SHAPE OF A LENS
PROCEDE ET DISPOSITIF POUR LA CORRECTION DE FORME D'UNE LENTILLE

(30) Priorität: 30.09.1992 DE 4232690
(43) Veröffentlichungstag der Anmeldung: 19.07.1995
(73) Patentinhaber: AESCULAP AG, 78532 Tuttlingen (DE)
(72) Erfinder: SCHRÖDER, Eckhard, D-90542 Eckental (DE)
(74) Vertreter: Böhme, Ulrich Hoeger, Stellrecht & Partner
(86) Internationale Anmeldenummer: EP9302577
(87) Internationale Veröffentlichungsnummer: WO9407422

(56) Entgegenhaltungen:
- EP-A- 0 417 952
- US-A- 4 729 372
- US-A- 4 941 093

## Beschreibung

Die Erfindung betrifft ein Verfahren zur Formkorrektur einer Linse, ausgenommen Formkorrektur am lebenden menschlichen oder tierischen Körper, bei dem man die Oberfläche der Linse durch eine Blende hindurch in bestimmten von der Blende freigegebenen Bereichen mit der Strahlung einer gepulsten Strahlungsquelle beaufschlagt und dabei beim Auftreffen jeden Strahlungspulses Material der Linse durch die Strahlungseinwirkung abträgt, wobei man die Strahlung in einem Strahlenbündel konzentriert, dessen Querschnitt beim Auftreffen auf der Linsenoberfläche kleiner ist als die von der Blende freigegebene Linsenoberfläche, und wobei man das Strahlenbündel so bewegt, daß durch nacheinander auf die Linsenoberfläche auftreffende Strahlungspulse die gesamte von der Blende freigegebene Linsenoberfläche bestrahlt wird.

Ferner betrifft die Erfindung eine Vorrichtung zur Durchführung dieses Verfahrens.

Mit dem beschriebenen Verfahren gelingt es, die Oberflächenform einer Linse, beispielsweise einer Kontaktlinse, durch sogenannte Photoablation so zu formen, daß eine optische Korrektur der Oberfläche erreicht werden kann. Herkömmlicherweise werden zu diesem Zweck als Strahlungsquellen Laser verwendet, und zwar gepulste Laser, die also in zeitlicher Abfolge wiederholt Strahlungsimpulse abgeben. Diese Strahlungsimpulse werden über der gewünschten Korrektur entsprechende Blenden auf die Linsenoberfläche gerichtet und tragen bei jedem Auftreffen eine sehr geringe Materialmenge von der Linsenoberfläche ab.

Es ist bekannt, bei derartigen Verfahren die gesamte von der Blende freigegebene Fläche der Linsenoberfläche von jedem Strahlungspuls vollständig bestrahlen zu lassen, d.h. der Querschnitt des auf die Blende gerichteten Strahlenbündels ist dann bei jedem Strahlungspuls größer als die von der Blende freigegebene Fläche. Dabei können sich jedoch Intensitätsprobleme ergeben, da die gesamte vom Laser erzeugte Strahlung unter Umständen über eine relativ große Fläche verteilt werden muß (US-A-4 941 093; US-A-4 729 372).

Andererseits ist es bekannt, die vom Laser erzeugte Strahlungsenergie in einem Strahlenbündel sehr kleinen Querschnitts zu konzentrieren, wobei dann dieses Strahlenbündel die von der Blende freigegebene Linsenoberfläche Scanner-ähnlich abtastet. Dabei ist es sehr schwierig, eine gleichmäßige Bestrahlung der gesamten Fläche zu erreichen, da sich die Positionen des Strahlungspulses exakt aneinander anschließen müssen (US-A-4 665 913).

Bei einem weiteren bekannten Verfahren wird die Strahlung in eine balkenförmige Fläche konzentriert, die schrittweise über die von der Blende freigegebene Fläche geführt wird. Dabei wird bei jeder Bestrahlung nur ein Teilbereich der freigegebenen Fläche bestrahlt, der Anschluß dieser Teilbereiche aneinander ist jedoch leichter zu bewerkstelligen (WO 92/00711).

Bei allen beschriebenen Verfahren wird im Endeffekt die von der Blende freigegebene Fläche an jeder Stelle von einem Strahlungspuls getroffen, es erfolgt also eine mehr oder weniger gleichmäßige Abtragung in dem gesamten von der Blende freigegebenen Bereich, deren Tiefe der Abtragung eines Strahlungspulses entspricht.

Wenn eine größere Abtragung gewünscht wird, ist es normalerweise notwendig, die Prozedur insgesamt zu wiederholen.

Es ist Aufgabe der Erfindung, eine unterschiedlich tiefe Abtragung in unterschiedlichen Bereichen der Linse zu ermöglichen.

Bei einer ersten bevorzugten Ausführung eines Verfahrens der eingangs beschriebenen Art wird diese Aufgabe erfindungsgemäß dadurch gelöst, daß man das Strahlenbündel zwischen aufeinanderfolgenden Strahlungspulsen nur so weit bewegt, daß sich die von nacheinander auf der Linsenoberfläche auftreffenden Strahlungspulsen getroffenen Bereiche der Linseroberfläche teilweise überlappen, und daß man die Größe der Überlappung der von nacheinander auf der Linsenoberfläche auftreffenden Strahlungspulsen getroffenen Bereiche der Linsenoberfläche bei Überstreichen einer Blendenöffnung variiert. Die von der Blendenöffnung freigegebene Linsenoberfläche wird dadurch unterschiedlich tief abgetragen, so daß durch die Überlappung der Abtragungsgrad gesteuert werden kann.

Bei einer anderen Ausführungsform, bei der die Blende um die optische Achse der Linse schrittweise rotiert wird, kann vorgesehen sein, daß man eine Blendenöffnung verwendet, die bezüglich der Drehachse der Blende nicht rotationssymmetrisch ist, und daß man die Größe der Überlappung der von nacheinander auf der Linsenoberfläche auftreffenden Strahlungspulsen getroffenen Bereiche der Linsenoberfläche bei unterschiedlichen Stellungen der Blende verschieden wählt. Bei jeder einzelnen Stellung der Blendenöffnung bleibt also die Überlappung konstant, so daß der gesamte von der Blende in einer Blendenstellung freigegebene Bereich der Linsenoberfläche gleichmäßig tief abgetragen wird. Dagegen kann durch die Variation der Überlappung bei unterschiedlichen Winkelstellungen der Blende eine unterschiedliche Abtragung in unterschiedlichen Winkelbereichen erzielt werden, so daß beispielsweise eine Korrektur von Myopie oder Hyperopie möglich wird, und zwar zusätzlich zu den unterschiedlichen Abtragungen in radialer Richtung, die sich aufgrund der Blendenform ergeben.

Im Prinzip kann ein solches Verfahren mit jeder Querschnittsform des Strahlenbündels durchgeführt werden, die eine insgesamt gleichmäßige Bestrahlung der von der Blende freigegebenen Fläche beim Abtasten ermöglicht, besonders günstig ist es aber, wenn der Querschnitt des Strahlenbündels die Form eines rechteckigen Balkens hat, der die von der Blende freigegebene Fläche in einer Richtung vollständig überdeckt, in der senkrecht dazu verlaufenden Richtung jedoch nur zu einem geringen Teil, und wenn man den Balken zwischen nacheinander folgenden Strahlungspulsen in dieser senkrecht verlaufenden Richtung bewegt. Die nacheinander auf die Linsenoberfläche auftreffenden Balken überlappen sich beim Überfahren der von der Blende freigegebenen Fläche einoder mehrfach, so daß eine sehr gleichmäßige Bestrahlung der Linsenoberfläche bei genau definierter Zahl der auftreffenden Strahlungspulse erreicht werden kann. Wichtig ist bei der Verwendung eines balkenförmigen Strahlenquerschnittes, daß die Linsenoberfläche mit einer relativ hohen Leistungsdichte bestrahlt werden kann, die auch bei relativ kleinen Lasern ausreicht, um die gewünschte Materialabtragung zu erreichen.

Es ist vorgesehen, daß die Blende erst dann in eine neue Position bewegt wird, wenn die gesamte von ihr freigegebene Fläche der Linsenoberfläche von Strahlungspulsen getroffen worden ist.

Dabei ist es vorteilhaft, wenn die Blende um die optische Achse der Linse schrittweise rotiert wird.

Der Erfindung liegt weiterhin die Aufgabe zugrunde, eine Vorrichtung zur Durchführung des beschriebenen Verfahrens zu schaffen. Derartige Vorrichtungen, die auch zur Formkorrektur am lebenden menschlichen oder tierischen Körper eingesetzt werden können, sind in den Patentansprüchen 6 und 7 beschrieben. Vorteilhafte Ausgestaltungen ergeben sich aus den Patentansprüchen 8 bis 10.

Die nachfolgende Beschreibung einer bevorzugten Ausführungsform der Erfindung dient im Zusammenhang mit der Zeichnung der näheren Erläuterung. Es zeigen:
- Fig. 1: eine schematische Ansicht einer Vorrichtung zur Formkorrektur einer Linse;
- Fig. 2: eine schematische Querschnittansicht der bestrahlten Linse in fünf aufeinanderfolgenden Bestrahlungsschritten bei einer Schrittweite, die der Hälfte des Strahlungsquerschnittes entspricht, und
- Fig. 3: eine Ansicht ähnlich Fig. 2 mit vier aufeinanderfolgenden Bestrahlungsschritten, wobei die Schrittweite nur ein Drittel des Strahlungsquerschnittes beträgt.

Die Erfindung wird im folgenden am Beispiel der Formung der Hornhaut eines Auges 1 erläutert, es versteht sich jedoch, daß auch andere Linsen in dieser Weise geformt werden können, beispielsweise Kontaktlinsen.

Vor der im folgenden allgemein als Linse 2 bezeichneten Hornhaut wird eine um die optische Achse der Linse 2 schrittweise verdrehbare Blende 3 angeordnet, die einen Durchlaß aufweist, der einen Teil der Oberfläche der Linse 2 freigibt, wobei sich der freiliegende Teil der Linse 2 von Position zu Position der Blende 3 ändert. Die Blende 3 wird durch einen Schrittmotor 4 schrittweise gedreht, der Motor 4 wird von einer Steuerung 5 über eine Leitung 6 gesteuert.

Diese Steuerung 5 steuert auch einen Laser 7, beispielsweise einen Excimer-Laser mit einer Wellenlänge von 193 nm, der von der Steuerung 5 gesteuert Strahlungspulse abgibt. Diese Strahlungspulse werden über einen Umlenkspiegel 8 auf die Blende 3 und durch deren offenliegende Teile hindurch auf die Oberfläche der Linse 2 gerichtet.

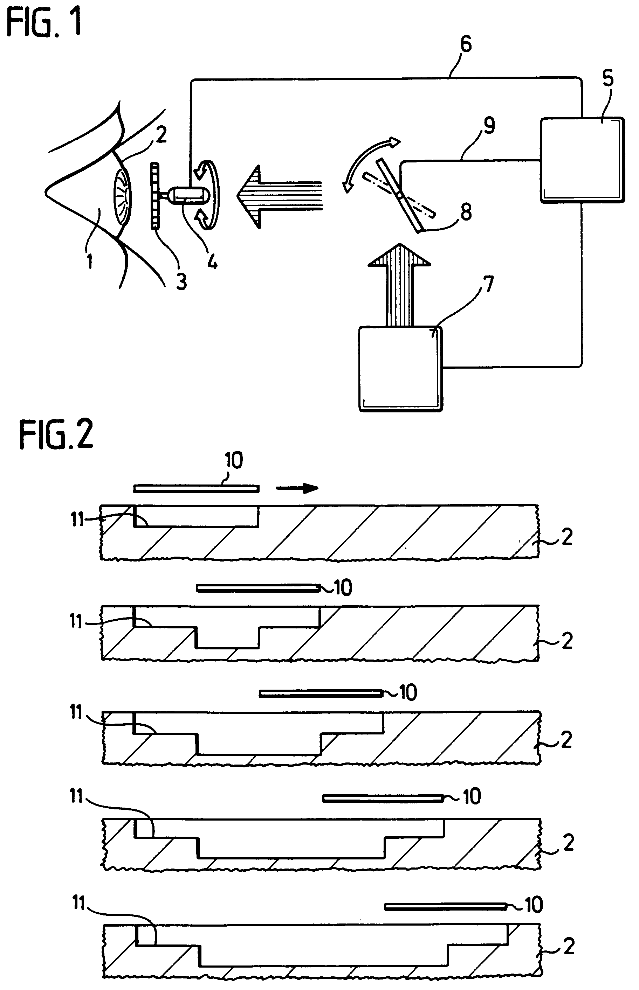

Der Umlenkspiegel 8 ist mit einem Antrieb versehen, der von der Steuerung 5 über eine weitere Leitung 9 angesteuert wird. Dadurch kann der Schwenkwinkel des Umlenkspiegels 8 verändert werden, in Fig. 1 sind zwei verschiedene Schwenkwinkel in ausgezogenen bzw. in strichpunktierten Linien dargestellt, wobei die Unterschiede im Ablenkungswinkel zur besseren Verdeutlichung in der Zeichnung übertrieben groß dargestellt sind.

In der Zeichnung nicht dargestellt sind optische Mittel, beispielsweise Zylinderlinsen, die den Strahlenquerschnitt der vom Laser 7 abgegebenen Strahlung so formen, daß der Strahlenquerschnitt die Form eines flachen langgestreckten Rechtecks oder Balkens aufweist. Dieser Balken hat Abmessungen, die in einer Richtung größer sind als die Öffnung in der Blende 3, in der senkrecht dazuliegenden Richtung jedoch deutlich kleiner, so daß bei jedem Strahlungspuls nur ein balkenförmiger Bereich der von der Blende 3 freigegebenen Oberfläche der Linse 2 von der Strahlung getroffen wird.

In Fig. 2 ist der Effekt dieses auf die Oberfläche der Linse 2 auftreffenden Strahlungspulses mit balkenförmigem Querschnitt schematisch dargestellt. Dabei wird die Fläche des vom Strahlungspuls eingenommenen Balkens durch ein langgestrecktes Rechteck 10 oberhalb der Oberfläche der Linse 2 angedeutet, wobei sich dieses Rechteck in der Zeichenebene nur über einen Teil der Linsenoberfläche erstreckt, die von der Blende 3 freigegeben ist, während senkrecht zur zeichenebene eine wesentlich größere Erstreckung vorgesehen ist, die die gesamte von der Blende freigegebene Öffnung überdeckt. Das Rechteck 10 symbolisiert also lediglich die Querschnittsausdehnung der auftreffenden Strahlungspulse in Ablenkungsrichtung des Strahlungsquerschnittes.

In der obersten Darstellung der Fig. 2 ist gezeigt, wie beim Auftreffen eines Strahlungspulses eine Vertiefung 11 in die Linsenoberfläche "gebrannt" wird, deren Ausdehnung im wesentlichen der Ausdehnung des Strahlenquerschnittes (symbolisiert durch das Rechteck 10) entspricht. Die Tiefe der Vertiefung 11 ergibt sich aus der Energie, die von einem Strahlungspuls auf die bestrahlte Fläche aufgebracht wird.

Gemäß der Erfindung wird der Strahlenquerschnitt nach jedem Strahlungspuls durch Verschwenkung des Umlenkspiegels 8 relativ zur Linse 2 verschoben, und zwar nur so weit, daß eine Teilüberdeckung erhalten bleibt. Im Falle der Fig. 2 beträgt der Verschiebeweg des Strahlenquerschnittes die halbe Breite des balkenförmigen Bestrahlungsbereiches, in Fig. 2 wird dies dadurch deutlich, daß das Rechteck 10 um seine halbe Länge verschoben wird.

Trifft in dieser Position erneut ein Strahlungspuls auf die Linsenoberfläche auf, so wird erneut in dem bestrahlten Bereich Material in einer Tiefe abgetragen, die der Tiefe der Vertiefung 11 entspricht. Durch die 50%ige Überdeckung ergibt sich im zentralen Bereich über eine Länge des halben Strahlungsquerschnittes eine Tiefe, die der doppelten Tiefe der Vertiefung 11 entspricht, wie dies in der zweiten Stufe der Fig. 2 dargestellt ist.

In den drei aufeinanderfolgenden Stufen der Fig. 2 ist der Strahlenquerschnitt jeweils um eine halbe Rechteckbreite weiter verschoben. Man erkennt, daß auf diese Weise die Oberfläche der Linse 2 so tief abgetragen wird, wie es der Energie von zwei auftreffenden Strahlungspulsen entspricht, lediglich im Randbereich ergibt sich eine Abstufung. Man erhält also auf diese Weise eine Abtragung, die die doppelte Tiefe einer normalen Abtragung aufweist, bei welcher die von der Blende freigegebene Fläche insgesamt einmal von Strahlungspulsen getroffen wird.

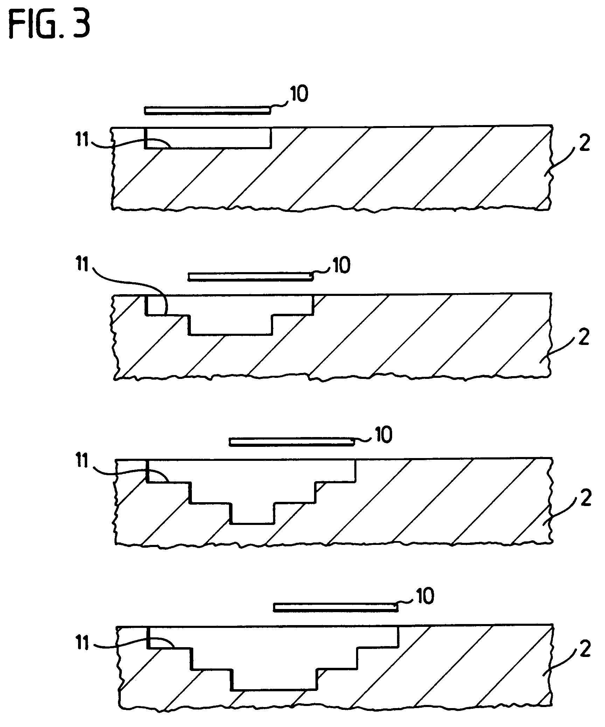

In Fig. 3 ist der entsprechende Vorgang für eine Verschiebung des Strahlenquerschnittes um eine geringere Verschiebestrecke beschrieben, nämlich bei einer Verschiebung um ein Drittel der Breite des Rechteckes 10. Man erkennt, daß auf diese Weise eine Abtragung erzielt werden kann, die der dreifachen Abtragung bei Einfachbestrahlung entspricht. Auch hier ergibt sich im Randbereich eine Abstufung.

Diese Steuerung ergibt natürlich die Möglichkeit, die Überdeckung bei dem von der Blende freigegebenen Bereich nicht über dessen gesamten Bereich konstant zu halten, sondern zu variieren, so daß auf diese Weise in Verschieberichtung des Strahlenquerschnittes eine unterschiedliche Profilierung erreicht werden kann. Dazu genügt es, über die Steuerung 5 die Schrittweite der Schwenkbewegung des Umlenkspiegels 8 zu variieren.

Mit der Steuerung ist es auch möglich, sphärische Korrekturen der Linsenoberfläche vorzunehmen, und zwar gleichzeitig mit asphärischen Korrekturen, wie beispielsweise dem Astigmatismus. Dies kann dadurch erreicht werden, daß die Blende schrittweise gedreht wird und daß bei jeder Blendenstellung die Überlappung durch die Steuerung unterschiedlich gewählt wird, so daß insgesamt eine unterschiedliche Abtragungsrate für unterschiedliche Winkelstellungen der Blende auftritt. Dies führt zu einer sphärischen Korrektur, während gleichzeitig die Blendenform für die asphärische Korrektur verantwortlich ist.
¹ The translator has presumed that 'Strahlungsbuendel' should read 'Strahlenbuendel' as in other instances.
² The translator has presumed that 'Strahlungsbuendel' should read 'Strahlenbuendel' asin other instances.

## Patentansprüche

1. Verfahren zur Formkorrektur einer Linse, ausgenommen Formkorrektur am lebenden menschlichen oder tierischen Körper, bei dem man die Oberfläche der Linse durch eine Blende hindurch in bestimmten von der Blende freigegebenen Bereichen mit der Strahlung einer gepulsten Strahlungsquelle beaufschlagt und dabei beim Auftreffen jeden Strahlungspulses Material der Linse durch die Strahlungseinwirkung abträgt, wobei man die Strahlung in einem Strahlenbündel konzentriert, dessen Querschnitt bei Auftreffen auf der Linsenoberfläche kleiner ist als die von der Blende freigegebene Linsenoberfläche, und wobei man das Strahlenbündel so bewegt, daß durch nacheinander auf die Linsenoberfläche auftreffende Strahlungspulse die gesamte von der Blende freigegebene Linsenoberfläche bestrahlt wird,
**dadurch gekennzeichnet**,
daß man das Strahlenbündel zwischen aufeinanderfolgenden Strahlungspulsen nur so weit bewegt, daß sich die von nacheinander auf der Linsenoberfläche auftreffenden Strahlungspulsen getroffenen Bereiche der Linsenoberfläche teilweise überlappen, und daß man die Größe der Überlappung der von nacheinander auf der Linsenoberfläche auftreffenden Strahlungspulsen getroffenen Bereiche der Linsenoberfläche bei Überstreichen einer Blendenöffnung variiert.

2. Verfahren zur Formkorrektur einer Linse, ausgenommen Formkorrektur am lebenden menschlichen oder tierischen Körper, bei dem man die Oberfläche der Linse durch eine Blende hindurch in bestimmten von der Blende freigegebenen Bereichen mit der Strahlung einer gepulsten Strahlungsquelle beaufschlagt und dabei beim Auftreffen jeden Strahlungspulses Material der Linse durch die Strahlungseinwirkung abträgt, wobei man die Strahlung in einem Strahlenbündel konzentriert, dessen Querschnitt bei Auftreffen auf der Linsenoberfläche kleiner ist als die von der Blende freigegebene Linsenoberfläche, und wobei man das Strahlenbündel so bewegt, daß durch nacheinander auf die Linsenoberfläche auftreffende Strahlungspulse die gesamte von der Blende freigegebene Linsenoberfläche bestrahlt wird,
**dadurch gekennzeichnet**,
daß man das Strahlenbündel zwischen aufeinanderfolgenden Strahlungspulsen nur so weit bewegt, daß sich die von nacheinander auf der Linsenoberfläche auftreffenden Strahlungspulsen getroffenen Bereiche der Linsenoberfläche teilweise überlappen, und daß man eine Blende mit einer Blendenöffnung verwendet, die bezüglich der Drehachse nicht rotationssymmetrisch ist, und daß man die Größe der Überlappung der von nacheinander auf der Linsenoberfläche auftreffenden Strahlungspulsen getroffenen Bereiche der Linsenoberfläche bei unterschiedlichen Stellungen der Blende verschieden wählt.

3. Verfahren nach Anspruch 1 oder 2, dadurch gekennzeichnet, daß der Querschnitt des Strahlenbündels die Form eines rechteckigen Balkens hat, der die von der Blende freigegebene Fläche in einer Richtung vollständig überdeckt, in der senkrecht dazu verlaufenden Richtung jedoch nur zu einem geringen Teil, und daß man den Balken zwischen nacheinanderfolgenden Strahlungspulsen in dieser senkrecht verlaufenden Richtung bewegt.

4. Verfahren nach Anspruch 1, 2 oder 3, dadurch gekennzeichnet, daß die Blende erst dann in eine neue Position bewegt wird, wenn die gesamte von ihr freigegebene Fläche der Linsenoberfläche von Strahlungspulsen getroffen worden ist.

5. Verfahren nach Anspruch 4, dadurch gekennzeichnet, daß die Blende um die optische Achse der Linse schrittweise rotiert wird.

6. Vorrichtung zur Formkorrektur einer Linse (2), welche die Oberfläche der Linse (2) durch eine Blende (3) hindurch in bestimmten von der Blende (3) freigegebenen Bereichen mit der Strahlung einer gepulsten Strahlungsquelle (7) beaufschlagt und dabei beim Auftreffen jeden Strahlungspulses Material der Linse (2) durch die Strahlungseinwirkung abträgt, wobei die Strahlung in einem Strahlenbündel konzentriert ist, dessen Querschnitt beim Auftreffen auf der Linsenoberfläche kleiner ist als die von der Blende (3) freigegebene Linsenoberfläche, und wobei das Strahlenbündel mittels steuerbarer optischer Mittel (8) so bewegt wird, daß durch nacheinander auf die Linsenoberfläche auftreffende Strahlungspulse die gesamte von der Blende (3) freigegebene Linsenoberfläche bestrahlt wird, dadurch gekennzeichnet, daß die steuerbaren optischen Mittel (8) das Strahlungsbündel zwischen aufeinanderfolgenden Strahlungspulsen nur so weit bewegen, daß sich die von nacheinander auf der Linsenoberfläche auftreffenden Strahlungspulsen getroffenen Bereiche der Linsenoberfläche teilweise überlappen, und daß die Steuerung (5) die Schrittweite der Bewegung der optischen Mittel (8) bei einer festen Stellung der Blende (3) variiert.

7. Vorrichtung zur Formkorrektur einer Linse (2), welche die Oberfläche der Linse (2) durch eine Blende (3) hindurch in bestimmten von der Blende (3) freigegebenen Bereichen mit der Strahlung einer gepulsten Strahlungsquelle (7) beaufschlagt und dabei beim Auftreffen jeden Strahlungspulses Material der Linse (2) durch die Strahlungseinwirkung abträgt, wobei die Strahlung in einem Strahlenbündel konzentriert ist, dessen Querschnitt beim Auftreffen auf der Linsenoberfläche kleiner ist als die von der Blende (3) freigegebene Linsenoberfläche, und wobei das Strahlenbündel mittels steuerbarer optischer Mittel (8) so bewegt wird, daß durch nacheinander auf die Linsenoberfläche auftreffende Strahlungspulse die gesamte von der Blende (3) freigegebene Linsenoberfläche bestrahlt wird, dadurch gekennzeichnet, daß die steuerbaren optischen Mittel (8) das Strahlungsbündel zwischen aufeinanderfolgenden Strahlungspulsen nur so weit bewegen, daß sich die von nacheinander auf der Linsenoberfläche auftreffenden Strahlungspulsen getroffenen Bereiche der Linsenoberfläche teilweise überlappen, und daß die Steuerung (5) die Schrittweite der Bewegung der optischen Mittel (8) zwischen verschiedenen Stellungen der Blende (3) variiert.

8. Vorrichtung nach Anspruch 6 oder 7, dadurch gekennzeichnet, daß der Querschnitt des Strahlenbündels die Form eines rechteckigen Balkens hat, der die von der Blende (3) freigegebene Fläche in einer Richtung vollständig überdeckt, in der senkrecht dazu verlaufenden Richtung jedoch nur zu einem geringen Teil, und daß die optischen Mittel (8) den Balken zwischen nacheinanderfolgenden Strahlungspulsen in dieser senkrecht verlaufenden Richtung bewegen.

9. Vorrichtung nach Anspruch 6, 7 oder 8, dadurch gekennzeichnet, daß die Steuerung (5) die Blende (3) erst dann in eine neue Position bewegt, wenn die gesamte von der Blende (3) freigegebene Fläche der Linsenoberfläche von Strahlungspulsen getroffen worden ist.

10. Vorrichtung nach einem der Ansprüche 6 bis 9, dadurch gekennzeichnet, daß die Steuerung (5) die Blende (3) um die optische Achse der Linse (2) schrittweise rotiert.

## Claims

1. A method of correcting the shape of a lens, excluding shape correction on live human or animal bodies, in which the surface of the lens is acted upon through a mask, in certain regions exposed by the mask, by the radiation of a pulsed radiation source and upon striking of each radiation pulse material of the lens is removed through the action of the radiation, the radiation being concentrated in a beam whose cross-section upon striking the lens surface is smaller than the lens surface exposed by the mask, and the beam being moved in such a manner that the entire lens surface exposed by the mask is irradiated by radiation pulses striking the lens surface one after another, characterised in that between successive radiation pulses, the beam is only moved to the extent that the lens surface regions affected by radiation pulses striking the lens surface one after another partially overlap, and in that the extent of overlap of the lens surface regions affected by radiation pulses striking the lens surface one after another is varied during sweeping of a mask opening.

2. A method of correcting the shape of a lens, excluding shape correction on live human or animal bodies, in which the surface of the lens is acted upon through a mask, in certain regions exposed by the mask, by the radiation of a pulsed radiation source and upon striking of each radiation pulse material of the lens is removed through the action of the radiation, the radiation being concentrated in a beam whose cross-section upon striking the lens surface is smaller than the lens surface exposed by the mask, and the beam being moved in such a manner that the entire lens surface exposed by the mask is irradiated by radiation pulses striking the lens surface one after another, characterised in that between successive radiation pulses, the beam is only moved to the extent that the lens surface regions affected by radiation pulses striking the lens surface one after another partially overlap, and in that a mask is used having a mask opening which is not rotationally symmetrical with regard to the axis of rotation, and in that differing extents of overlap of the lens surface regions affected by radiation pulses striking the lens surface one after another are selected for different positions of the mask.

3. A method in accordance with Claim 1 or 2, characterised in that the cross-section of the beam has the form of a rectangular bar which in one direction completely covers the area exposed by the mask, but in the direction perpendicular thereto only covers a small part of this area, and in that the bar is moved in this perpendicular direction between successive radiation pulses.

4. A method in accordance with Claim 1, 2 or 3, characterised in that the mask is only moved into a new position when the entire lens surface area which it exposes has been affected by radiation pulses.

5. A method in accordance with Claim 4, characterised in that the mask is rotated in a stepwise manner about the optical axis of the lens.

6. A device for correcting the shape of a lens (2), which acts upon the surface of the lens (2) through a mask (3), in certain regions exposed by the mask (3), by the radiation of a pulsed radiation source (7) and upon striking of each radiation pulse removes material of the lens (2) through the action of the radiation, the radiation being concentrated in a beam whose cross-section upon striking the lens surface is smaller than the lens surface exposed by the mask (3), and the beam being moved by means of controllable optical means (8) in such a manner that the entire lens surface exposed by the mask (3) is irradiated by radiation pulses striking the lens surface one after another, characterised in that between successive radiation pulses, the beam¹ is only moved by the controllable optical means (8) to the extent that the lens surface regions affected by radiation pulses striking the lens surface one after another partially overlap, and in that the control device (5) varies the step width of the movement of the optical means (8), the mask (3) being in a fixed position.

7. A device for correcting the shape of a lens (2), which acts upon the surface of the lens (2) through a mask (3), in certain regions exposed by the mask (3), by the radiation of a pulsed radiation source (7) and upon striking of each radiation pulse removes material of the lens (2) through the action of the radiation, the radiation being concentrated in a beam whose cross-section upon striking the lens surface is smaller than the lens surface exposed by the mask (3), and the beam being moved by means of controllable optical means (8) in such a manner that the entire lens surface exposed by the mask (3) is irradiated by radiation pulses string the lens surface one after another, characterised in that between successive radiation pulses, the beam² is only moved by the controllable optical means (8) to the extent that the lens surface regions affected by radiation pulses striking the lens surface one after another partially overlap, and in that the control device (5) varies the step width of the movement of the optical means (8) between different positions of the mask (3).

8. A device in accordance with Claim 6 or 7, characterised in that the cross-section of the beam has the form of a rectangular bar which in one direction completely covers the area exposed by the mask (3), but in the direction perpendicular thereto only covers a small part of this area, and in that between successive radiation pulses, the bar is moved in this perpendicular direction by the optical means (8).

9. A device in accordance with Claim 6, 7 or 8, characterised in that the control device (5) only moves the mask (3) into a new position when the entire lens surface area exposed by the mask (3) has been affected by radiation pulses.

10. A device in accordance with any one of Claims 6 to 9, characterised in that the control device (5) rotates the mask (3) about the optical axis of the lens (2) in a stepwise manner.

## Revendications

1. Procédé pour corriger la forme d'une lentille, à l'exception d'une correction de forme sur un corps vivant, humain ou animal, dans le cas duquel on intervient sur la surface de la lentille, à travers un diaphragme, sur des zones déterminées, libérées par le diaphragme, avec l'irradiation d'une source pulsatoire d'irradiation et, lors de l'impact de chaque impulsion d'irradiation, on y enlève de la matière de la lentille par l'action de l'irradiation, procédé dans lequel on concentre l'irradiation dans un faisceau de rayons dont la section, lors de son impact sur la surface de la lentille est inférieure à la surface de la lentille libérée par le diaphragme, et dans lequel on déplace le faisceau de rayons de façon que la totalité de la surface de la lentille libérée par le diaphragme soit irradiée par des impulsions d'irradiation atteignant successivement la surface de la lentille,
caractérisé par le fait
que l'on ne déplace le faisceau de rayons entre des impulsions d'irradiation successives que suffisamment pour que les zones de la lentille atteintes par les impulsions d'irradiation qui atteignent successivement la surface de la lentille se recouvrent partiellement, et que, lors du balayage effectué par une ouverture du diaphragme, on fait varier la valeur du recouvrement des zones de la surface de la lentille atteintes par des impulsions d'irradiation atteignant successivement la surface de la lentille.

2. Procédé pour corriger la forme d'une lentille, à l'exception d'une correction de forme sur un corps vivant, humain ou animal, dans le cas duquel on intervient sur la surface de la lentille, à travers un diaphragme, sur des zones déterminées, libérées par le diaphragme, avec l'irradiation d'une source pulsatoire d'irradiation et, lors de l'impact de chaque impulsion d'irradiation, on y enlève de la matière de la lentille par l'action de l'irradiation, procédé dans lequel on concentre l'irradiation dans un faisceau de rayons dont la section, lors de son impact sur la surface de la lentille est inférieure à la surface de la lentille libérée par le diaphragme, et dans lequel on déplace le faisceau de rayons de façon que la totalité de la surface de la lentille libérée par le diaphragme soit irradiée par des impulsions d'irradiation atteignant successivement la surface de la lentille,
caractérisé par le fait
que l'on ne déplace le faisceau de rayons entre des impulsions d'irradiation successives que suffisamment pour que les zones de la lentille atteintes par les impulsions d'irradiation qui atteignent successivement la surface de la lentille se recouvrent partiellement, et que l'on emploie un diaphragme avec une ouverture de diaphragme qui ne présente pas de symétrie de rotation par rapport à l'axe de rotation et que, pour différentes positions du diaphragme, on choisit différente la valeur du recouvrement des zones de la surface de la lentille atteintes par des impulsions d'irradiation atteignant successivement la surface de la lentille.

3. Procédé selon la revendication 1 ou 2, caractérisé par le fait que la section du faisceau de rayons a la forme d'une poutre rectangulaire qui recouvre complètement dans une direction la surface libérée par le diaphragme mais qui n'en recouvre qu'une petite partie dans la direction perpendiculaire et que c'est dans cette direction perpendiculaire que l'on déplace la poutre entre des impulsions d'irradiation successives.

4. Procédé selon la revendication 1, 2 ou 3, caractérisé par le fait que l'on n'amène le diaphragme à une nouvelle position que lorsque la totalité de la surface de la lentille qu'il libère a été atteinte par des impulsions d'irradiation.

5. Procédé selon la revendication 4, caractérisé par le fait que le diaphragme est entraîné en rotation pas à pas autour de l'axe optique de la lentille.

6. Dispositif pour corriger la forme d'une lentille (2), qui intervient sur la surface de la lentille (2), à travers un diaphragme (3), sur des zones déterminées, libérées par le diaphragme (3), avec l'irradiation d'une source pulsatoire d'irradiation (7) et, lors de l'impact de chaque impulsion d'irradiation, y enlève de la matière de la lentille (2) par l'action de l'irradiation, dispositif dans lequel l'irradiation est concentrée en un faisceau de rayons dont la section, lors de l'impact sur la surface de la lentille, est inférieure à la surface de la lentille libérée par le diaphragme (3), et dans lequel le faisceau de rayons se déplace, grâce à des moyens optiques pilotables (8), de façon que la totalité de la surface de la lentille libérée par le diaphragme (3) soit irradiée par des impulsions d'irradiation atteignant successivement la surface de la lentille, caractérisé par le fait que les moyens optiques pilotables (8) ne déplacent le faisceau de rayons entre des impulsions d'irradiation successives que suffisamment pour que les zones de la surface de la lentille atteintes par des impulsions d'irradiation atteignant successivement la surface de la lentille se recouvrent partiellement, et que, pour une position fixe du diaphragme (3), l'organe de commande (5) fait varier le pas du mouvement des moyens optiques (8).

7. Dispositif pour corriger la forme d'une lentille (2), qui intervient sur la surface de la lentille (2), à travers un diaphragme (3), sur des zones déterminées, libérées par le diaphragme (3), avec l'irradiation d'une source pulsatoire d'irradiation (7) et, lors de l'impact de chaque impulsion d'irradiation, y enlève de la matière de la lentille (2) par l'action de l'irradiation, dispositif dans lequel l'irradiation est concentrée en un faisceau de rayons dont la section, lors de l'impact sur la surface de la lentille, est inférieure à la surface de la lentille libérée par le diaphragme (3), et dans lequel le faisceau de rayons se déplace, grâce à des moyens optiques pilotables (8), de façon que la totalité de la surface de la lentille libérée par le diaphragme (3) soit irradiée par des impulsions d'irradiation atteignant successivement la surface de la lentille, caractérisé par le fait que les moyens optiques pilotables (8) ne déplacent le faisceau de rayons entre des impulsions d'irradiation successives que suffisamment pour que les zones de la surface de la lentille atteintes par des impulsions d'irradiation atteignant successivement la surface de la lentille se recouvrent partiellement, et que l'organe de commande (5) fait varier le pas du mouvement des moyens optiques (8) entre différentes positions du diaphragme (3).

8. Dispositif selon la revendication 6 ou 7, caractérisé par le fait que la section du faisceau de rayons a la forme d'une poutre rectangulaire qui recouvre complètement dans une direction la surface libérée par le diaphragme (3) mais qui n'en recouvre qu'une petite partie dans la direction perpendiculaire, et que c'est dans cette direction perpendiculaire que les moyens optiques (8) déplacent la poutre entre des impulsions d'irradiation successives.

9. Dispositif selon la revendication 6, 7 ou 8, caractérisé par le fait que l'organe de commande (5) n'amène le diaphragme (3) dans une nouvelle position que lorsque la totalité de la surface de la lentille libérée par le diaphragme (3) a été atteinte par des impulsions d'irradiation.

10. Dispositif selon l'une des revendications 6 à 9, caractérisé par le fait que l'organe de commande (5) entraîne en rotation pas à pas le diaphragme (3) autour de l'axe optique de la lentille (2).
